Europäisches Patentamt

European Patent Office

Office européen des brevets

(19)

(11) Publication number: **0 218 400 B1**

# EUROPEAN PATENT SPECIFICATION

(12)

(45) Date of publication of the patent specification:
05.12.90

(51) Int. Cl.⁵: **A61K 49/00, C12Q 1/18, C12N 11/04, G01N 33/48**

(21) Application number: 86307187.4

(22) Date of filing: 18.09.86

(54) Assay methods for chemotherapeutic agents.

(30) Priority: 18.09.85 US 777170
09.09.86 US 903978

(43) Date of publication of application:
15.04.87 Bulletin 87/16

(45) Publication of the grant of the patent:
05.12.90 Bulletin 90/49

(84) Designated Contracting States:
AT BE CH DE FR GB IT LI LU NL SE

(56) References cited:
EP-A- 0 048 109
WO-A-82/00660
GB-A- 2 094 833
GB-A- 2 144 543
US-A- 4 391 909
US-A- 4 411 990
US-A- 4 564 517

BIOLOGICAL ABSTRACTS, vol. 58, 15th
November 1974, page 6037, no. 56133, Philadelphia, PA.,
US; V.F.GARRY, Jr. et al.: "Use of the cytograf to
determine cytotoxicity in fibroblast cell cultures" &
ONCOLOGY (BASEL) 29(5): 429-438. ILLUS. 1974

(73) Proprietor: DAMON BIOTECH, INC., 119 Fourth Avenue,
Needham Heights Massachusetts 02194(US)

(72) Inventor: Gorelik, Elieser, 230 North Craig Street,
Pittsburgh Pennsylvania 15213(US)
Inventor: Jarvis, Allan P., Jr., 2 Summit Place,
Newburyport Massachusetts 01950(US)

(74) Representative: Harvey, David Gareth et al, Graham
Watt & Co. Riverhead, Sevenoaks Kent TN13 2BN(GB)

**Description**

This invention relates to cell culturing and, in particular, assay methods for measuring cytotoxicity.

It is known that one patient with a cancer or other cell abnormality may respond differently than another patient to a given drug therapy even if their diseases are similar. Moreover, in the search for new chemotherapeutic agents, it is not uncommon for drugs of nearly identical structure to have very different effects in combating cancerous or other abnormal cells. For these reasons, predictive assay techniques similar to microbial sensitivity assays, would be great assistance in selecting effective chemotherapy for individual cases and in screening new drugs for chemotherapeutic activity.

One conventional approach to predictive and screening assays for chemotherapy involves the growth of cell cultures on soft agar. See, for examples, U.S. Patent 4,411,990 entitled "Bioassay of Human Tumor Stem Cells" by Salmon et al., issued on October 25, 1983; Salmon et al, "Quantitation of Differential Sensitivity of Human Stem Cells to Anti-Cancer Drugs," Vol. 298 New England Journal of Medicine pp. 1321-1327 (1978); Buick et al., "Development of an Agar-Methyl Cellulose Clonogenic Assay for Cells in Transitional Cell Carcinoma of the Human Bladder," Vol. 39 Cancer Research pp. 5051-5056 (1979); and Von Hoff et al., "Direct Cloning of Human Malignant Melanoma in Soft Agar Culture," Vol. 50 Cancer pp. 695-701 (1982). These articles describe in detail the technique of cloning cells from biopsy specimens in soft agar after brief exposures to anti-cancer drugs.

Various difficulties limit the usefulness of agar-culture studies for predicting the effectiveness of cytotoxic agents against abnormal cells. Only a fraction of biopsied cancer cells grow in soft agar and tumor cells are able to grow in agar usually have low cloning efficiencies. Thus, for statistically significant results comparing different drugs at different doses, large numbers of cells are required. Moreover, agar techniques typically limit drug exposure to a relatively brief period (e.g., one hour) prior to plating while the cell is suspended in a physiological solution. Additionally, some chemotherapeutic drugs do not work unless acted upon by enzymes or other substances found in the patients body, and it can often be difficult to distinguish between colonies proliferating on agar and agglomeration of incompletely dissociated cells from the biopsy sample. Hence, neither the exposure technique nor the subsequent growth in agar accurately mimic in vivo conditions.

Another approach to assaying the effects of chemotherapeutic agents involves the induction of tumors in mice or other laboratory animals. This technique is typically employed to screen new compounds for chemotherapeutic effects. After the animal is exposed to a known tumor-inducing cell line, it is treated with the chemotherapeutic agent and the effects of the agent on tumor survival and growth are observed. There is often only a limited correlation between the results on a particular animal and the response of the various types of human cancers to the same drug. Moreover, assays of this type typically require very large numbers of expensive nude (i.e., immunodeficient) mice and long time periods for observation, maintenance and handling of the experimental animals.

Accordingly, there exists a need for better method of performing cytotoxicity assays. Assays that could assist medical practitioners in selecting individualized anti-cancer drug regimes would satisfy a long-felt need in the field of cancer treatment. Without such individualized protocols, practitioners are forced to rely upon past experience or reports on similar cell disorders or trial-and-error procedures. Moreover, in the continuing efforts to find new and better chemotherapeutic agents, an assay that could accurately mimic in vivo conditions would also satisfy a long-felt need in the field.

A new method of assaying cells for sensitivity to therapeutic agents is disclosed herein consisting of the steps of: encapsulating the cells within a semipermeable membrane, inoculating the encapsulated cells into a living host animal; exposing the cells to the therapeutic agent before or after inoculation; retrieving the microcapsules from the host animal; and, measuring changes in the encapsulated cells to provide an indication of cytotoxic effects. The inoculated, encapsulated cells provide an ideal environment that can mimic invivo growth conditions under which the efficacy of various of chemotherapeutic agents can be tested.

The invention permits both predictions of optimal treatment protocols for individual cases and the screening of new substances of unknown efficacy. It has been demonstrated that anti-cancer drugs reach the inoculated, encapsulated cells within host animals and have cytotoxic effects. Moreover, the results correlate well with the known effects of particular chemotherapeutic agents on particular cell lines. The present invention is applicable not only to the screening of anti-cancer effects of various chemical and natural substances but also to biological response modifiers (e.g. interferons, interleukins, retinoic acid, tumor necrosis factors, etc.), monoclonal antibodies and such antibodies conjugated with anti-tumor toxins. The assay is useful in determining both cytotoxic and cytostatic effects of experimental protocols.

In one preferred method, a biopsy sample is taken and mechanically diced into smaller fragments, which are suspended in a medium and treated with enzymes to separate the malignant cells. The tumor cells can be further purified by the various known techniques. Once the tumor cells have been isolated, they are encapsulated as described below and the semipermeable capsules thus formed are implanted, preferably in the peritoneal cavity, in a host animal. The semipermeable microcapsules prevent the tumor cells from being destroyed by the animal's immune response, but allow nutrients and other humoral factors, including the chemotherapeutic agent, to enter inside the capsule and reach the tumor cells. Each implanted animal receives either the agent under study or a placebo. After several days, the microcapsules are retrieved and examined. Cell death within the microcapsules, or a reduction in cell numbers in comparison to those capsules retrieved from the control animals, provides a quantitative measurement of the effectiveness of the particular chemotherapeutic agent. The methods disclosed herein also permit the testing of the effects, the results of prolonged exposure, different combinations and programmed schedules of drugs.

In one embodiment, the cells are exposed to the cytotoxic agent in vitro at various concentrations prior to innoculating the host animal with such encapsulated cells. In another preferred embodiment, the cells are encapsulated and inoculated into the host animal and then the cytotoxic agent is administered in vivo in single or multiple doses by one or another administration route. The encapsulated cells are cultured within the host animal (e.g., for 7-14 days) and then retrieved for analysis.

According to the invention, cancerous tissue or cells are encapsulated by following the methods disclosed in U.S.-A-4,352,883; U.S.-A-4,391,909; U.S.-A-4,409,331 and U.S.-A-4,495,288. The said patents are assigned to the present Applicant.

In accordance with the teachings of the above patents, water-soluble substances which are physiologically compatible with living tissue are used to form a shape-retaining, coherent mass, as a "temporary capsule" or protective barrier layer about cancer cells. One preferred gel-forming substance is sodium alginate. The temporary capsule is treated to deposit a more permanent semipermeable membrane about the cells without damage to the cells. The gel forming substance is added to the tissue culture medium which also contains the cancer cells, serum components (if required), and collagen or another high molecular weight, water-dispensable material (if required) to act as an anchoring substrate. The concentration of the material employed as a substrate can be within the range of about 10 ug/ml to about 1 mg/ml but is preferably on the order of 100-500 ug/ml. Polylysine and polyornithine are two preferred polymers for membrane formation.

The various therapeutic or chemical agents which can be tested according to the invention for effectiveness on individual cell cultures include: adriamycins, mitomycins, actinomycins, neomycins, vincristine, vinblastine, chlorabucil, cis-platinum, 6-mercapto purine, methotrexate, cyclophosphamide, melphalen, carmustine, methyl ester DOPA, BCNU, DTIC, 5-fluorouracil, m-AMSA, mitoxantrone, methyl GAG, acivicin, thymidine, hormones, antibodies, prostaglandins and lymphokines as well as X-rays or other agents as they become available. In the preferred embodiments, the chemotherapeutic agents are administered by intravenous injection, intraperitoneal injection, subcutaneous injection or orally, depending upon the agent, to further mimic human treatment protocols.

The encapsulated cells can be retrieved from the animal host after sacrificing the animal by washing the peritoneal cavity and aspirating the capsules from the wash solution. Alternately, the capsules can be initially placed in a biocompatible chamber, sack or tube and then implanting into the peritoneal cavity surgically. In some instances, the implanted chamber can then be retrieved without sacrificing the host.

Once the capsules are retrieved, the capsule membranes are disrupted and the cells suspended for counting. In one preferred technique, the cells are stained and then counted using a hemocytometer. Other cell sorting and cell counting techniques can also be employed. For example, metabolizable vital dyes, such as 2-(p-iodophenyl-3 (p-nitrophenyl)-5-phenyl terazolium chloride (INT) can be used to quantize the number of viable tumor cells. Other techniques also include total tumor protein (or DNA) analysis and computerized imaging of the tumor mass.

The invention will now be described by way of example in connection with certain embodiments.

In the following examples, the encapsulation techniques of the above-referenced Damon patents were employed to prepare microcapsules containing cancer cells. In accordance with the techniques, a solution containing the cancer cells, gel-forming substance and supplements is formed into droplets and is immediately rendered water-insoluble and gelled, at least in a surface layer. Thereafter, the shape-retaining temporary capsules are provided with a more permanent membrane. The capsule interior is reliquified after formation of the permanent membrane. This is done by re-establishing the conditions in the medium at which the gel-forming material is soluble.

The material used to form the temporary capsules may be any non-toxic, water-soluble material which, by a change in ionic environment or concentration, can be converted to a shape-retaining mass. The material should also contain plural, easily ionized anionic moities, e.g., carboxyl groups, which can react by salt formation with polymers containing plural cationic groups. Use of this type of material enables one to deposit a permanent membrane of a selected upper limit of permeability (generally no greater than 100,000 to 150,000 daltons) without difficulty in surface layers of the temporary capsule.

The presently preferred materials for forming the temporary capsule are acidic, water-soluble, natural or synthetic polysaccharide gums. Such materials are commercially available. They are typically extracted from vegetable matter and are often used as additives to various foods. Sodium alginate is the presently preferred water-soluble gum. Alginate in the molecular weight range of 150,000+ daltons may be used, but because of its molecular dimensions and viscosity will usually be unable to permeate the finally formed capsule membranes. Lower molecular weight alginate, e.g., 50,000-80,000 daltons, is more easily removed from the intracapsular volume by diffusion through a membrane of sufficient porosity and is therefore preferred. Other usable gums include acidic fractions of guar gum, carrageenan, pectin, tragacanth gum, or xanthan gum.

These materials comprise glycoside-linked saccharide chains. Their free acid groups are often present in the alkali metal ion form, e.g., sodium form. If a multivalent ion such as calcium or strontium is exchanged for the alkali metal ion, the water-soluble polysaccharide molecules are "cross-linked" to form a water-soluble, shape-retaining gel which can be resolubilized on removal of the ions by ion exchange or via a sequestering agent. While essentially any multivalent ion which can form a salt with the acidic gum is operable, it is preferable that physiologically compatible ions, e.g., calcium,, be employed. This tends to preserve the tissue in the living state. Other multivalent cations can be used. Magnesium ions are ineffective in gelling sodium alginate.

A typical solution composition comprises 2 ml of a tumor cell suspension in its medium (with an anchoring substrate if desired) and 10 ml of a one or two percent solution of gum in physiological saline. When employing sodium alginate, a 1.0 to 1.5 percent solution has been used with success. Collagen or another high molecular weight water-dispensable protein or polypeptide, either natural or synthetic, may be included in the cell culture, and will be confined within the intracapsular volume of the finally formed capsules. The average diameter of the material to be encapsulated can vary widely between a few micrometers to about a millimeter. However, best results are achieved with capsules of a size in the range of 100-500 micrometers.

If a polymer having plural catonic groups is employed, e.g., polylysine, the cationic groups react with anionic sites in the water-soluble gum to form a substantially water-insoluble matrix interwined with the gum. Preferred concentrations for such materials are on the order of 10-500 ug per ml of suspension (including gum solution).

In the next step of the encapsulation process, the gum solution containing the tissue is formed into droplets of a desired size, and the droplets are immediately gelled to form shape-retaining spherical or spheroidal masses. The drop formations may be conducted as follows: A tube containing an aqueous solution of multivalent cations, e.g., 1.5% $CaCl_2$ solution, is fitted with a stopper which holds a drop forming apparatus. The apparatus consists of a housing having an upper air intake nozzle and an elongate hollow body friction fitted into the stopper. A 10 ml syringe equipped with a stepping pump is mounted atop the housing with, e.g., a 0.01 inch (0.254 mm) I.D. Teflon coated needle passing through the length of the housing. The interior of the housing is designed such that the tip of the needle is subjected to a constant laminar air flow which acts as an air knife. In use, with the syringe full of solution containing the material to be encapsulated, the stepping pump is actuated to incrementally force droplets of solution from the tip of the needle.

Each drop is "cut off" by the air stream and falls approximately 2.5 cm into the $CaCl_2$ solution where it is immediately gelled by absorption of calcium ions. The distance between the tip of the needle and the surface of the $CaCl_2$ solution is great enough, in this instance, to allow the sodium alginate/cell suspension to assume the most physically favorable shape: a sphere (maximum volume for minimum surface area). Air within the tube bleeds through an opening in the stopper. This results in "cross-linking" of the gel and in the formation of a high viscosity shape-retaining protective temporary capsule containing the suspended tissue and its medium. The capsules collect in the solution as a separate phase and may be separated by aspiration.

After the temporary capsules are formed, a semipermeable membrane is deposited about the surface of the capsules by "cross-linking" surface layers. This may be effected by subjecting the gelled temporary capsules to an aqueous solution of a polymer containing cationic groups reactive with anionic functionalities in the gel molecules. Polymers containing acid reactive groups such as free imine of amine groups are preferred. In this situation, the polysaccharide gum is cross-linked by interaction (salt bond formation) between the carboxyl groups and the amine or imine groups. Permeability can be controlled within limits by selecting the molecular weight of the cross-linking polymer used and by regulating the concentration of the polymer solution and the duration and temperature of exposure. A solution of polymer having a low molecular weight, in a given time period, will penetrate further into the temporary capsules than will a high molecular weight polymer. The degree of penetration of the cross-linker has been correlated with the resulting permeability. In general, the higher the molecular weight and the less penetration, the larger the pore size. Broadly, polymers within the molecular range of 3,000 to 100,000 daltons or greater may be used, depending on the duration of the reaction, the concentration of the polymer solution, and the degree of permeability desired. One successful set of reaction conditions, using polylysine of average molecular weight of about 35,000 daltons, involved reaction for two minutes, with stirring, of a physiological saline solution containing 0.0167 percent polylysine. This results in membranes having an upper permeability limit of about 100,000 daltons. Optimal reaction conditions suitable for controlling permeability in a given system can readily be determined empirically in view of the foregoing guidelines. Using this method it is possible to set the upper permeability limit of the membranes at a selected level generally below about 150,000 daltons.

Examples of suitable cross-linking polymers include proteins and polypeptides, either natural or synthetic, having free amino or imino groups, polyethyleneamines, polyethyleneimines, and polyvinylamines. Polylysine, in both the D and L forms, has been used with success. Proteins such as polyarginine, polycitrulline, or polyornithine are also operable. Polymers in the higher range of positive charge density, e.g., polyvinylamine, vigorously adhere to the anionic groups of the gel molecules to form stable membranes, but the membranes are somewhat difficult to disrupt.

Treatment with a dilute solution of gum or a zwitterionic buffer will tie up free amino groups on the surface of the capsules which otherwise may impart to the capsules a tendency to clump.

At this point in the encapsulation, capsules may be collected which comprise a semipermeable membrane surrounding a gelled solution of gum, cell-type compatible culture medium, cells, and an internal matrix of collagen or another anchorage substrate. Since mass transfer should be promoted within the capsules and across the membranes, it is preferred to reliquify the gel to its water-soluble form. This may be done by re-establishing the conditions under which the gum is a liquid, e.g., removing the calcium or other multi-functional cations from the interior gel. The medium in the capsule can be resolubilized simply by immersing the capsules in phosphate buffered saline, which contains alkali metal ions and hydrogen ions. Monovalent ions exchange with the calcium or other multi-functional ions with the gum when the capsules are immersed in the solution with stirring. Sodium citrate solutions may be used for the same purpose, and serve to sequester the divalent ions.

The encapsulated tumors are preferably incubated at 37° C in medium for 1 to 2 hours. A 25 percent (v/v) suspension can then be prepared (preferably without bovine serum). The suspension can be inoculated in the peritoneal cavity of the host animal (i.e., with a 16 gauge needle).

In the experiments that follow, the drugs and other agents tested were administered to the host animal by injection at the various times called for in the protocol being tested. At various periods after treatment, the mice were killed by cervical dislocation. The peritoneal cavity of each mouse was washed with PBS or medium and floating microcapsules were aspirated using a large bore plastic Pasteur pipette. Microcapsules were transferred to 15 ml tubes and washed 3 to 4 times with PBS to remove host peritoneal cells. The washed microcapsules were aspirated into 1 ml serological pipettes, allowed to sediment, and the volume of packed microcapsules was determined. Microcapsules were then transferred to a glas dounce homogenizer (7 ml volume) and disrupted with 3 strokes of an A-tolerance plumger. Cells suspensions were filtered through nylon mesh to remove the disrupted membranes. The suspensions containing tumor cells were centrifuged and the pellet was resuspended in 0.25 to 0.5 ml of complete medium. After mixing equal volumes of cell suspension and trypan blue, viable cell counts were determined using a hemocytometer.

The number of tumor cells per 1 ml of packed microcapsules was determined as follows:

$$a = \frac{b}{c}$$

where b = total number of tumor cells in the suspension, and
c = total volume of microcapsules used for the preparation of this suspension.

By comparing the number of tumor cells in 1 ml of microcapsules derived from non-treated and drug-treated mice, the percent inhibition of tumor cell growth was calculated:

$$\% \text{ inhibition} = \frac{a_c - a_e}{a_c} \times 100$$

where $a_c$ = number of tumor cells from the non-treated control microcapsules, and $a_e$ = number of tumor cells from the drug treated microcapsules.

The invention will be further illustrated in connection with following examples.

Example 1

Human T cell lymphoma (MOLT-4) cells were encapsulated according to the procedures described above and incubated in vitro in RPMI I640 medium supplemented with 10 percent fetal calf serum, glutamine and antibiotics for one hour of 37°C. During incubation they were exposed to various concentrations of 1,3-B (2-chloroethyl)-1-nitrosourea (BCNU), 5-fluorouracil (5-FU) and adriamycin. The microcapsules were then washed and implanted into the peritoneal cavity (i.p.) of C57BL/6 mice (Animal Production Area NCI-Frederick Cancer Research Facility) using #18-14 needles. After seven days the mice were sacrificed and their peritoneal cavities were washed with PBS and aspirated to collect the microcapsules in a Pasteur pipette. The microcapsules were washed several times from the host peritoneal cells. The microcapsules from the individual mice were counted by transferring them into wells of a 96 well microplate. Using an inverted microscope, the numbers of microcapsules in each well were counted. Each well was washed out and microcapsules were transferred into the glass homogenizer.

Microcapsules were then disrupted, and the suspension was filtered, centrifugated and reconstituted with PBS. The concentration and total number of cells were counted using a hemocytometer. The results of the in vitro treatment with BCNU, 5-FU and adriamycin are shown below in Table 1.

Table 1

| Influence of in vitro pretreatment with BCNU, 5-Fu and Adriamycin on in vivo growth of human lymphoma cells Molt-4. | | |
|---|---|---|
| | No. of tumor cells | |
| Treatment | per capsule | % inhibition |
| None | $7.5 \times 10^4$ | |
| BCNU in vitro | | |
| 100 µg/ml | $0.1 \times 10^4$ | 98 |
| 25 µg/ml | $1 \times 10^4$ | 86 |
| 6 µg/ml | $1 \times 10^4$ | 86 |
| None | $5 \times 10^3$ | |
| 5-Fu in vitro | | |
| 100 µg/ml | $10^2$ | 98 |
| 25 µg/ml | $3 \times 10^3$ | 40 |
| None | $5 \times 10^3$ | |
| Adriamycin in vitro | | |
| 10 µg/ml | 10 | 100 |
| 2.5 µg/ml | $2 \times 10^3$ | 60 |

Example 2

MOLT-4 lymphoma cells were also used in an in vivo assay. As before, the MOLT-4 cells were encapsulated and transplanted i.p. into C57BL/6 mice. On day 1 following the transplantation of the encapsulated cells, the mice were treated i.v. intravenous (i.v.) with BCNU, adriamycin or cyclophosphamide (CY). This approach allowed the testing of cyclophosphamide (CY) which is inefficient in vitro because it must be metabolized to obtain an active cytotoxic derivative. Ten days after transplantation, the encapsulated cells were retrieved and the tumor cells contained therein were counted. The results indicated the drugs were able to reach the encapsulated cells even when administered i.v. and that adriamycin and CY were more effective than BCNU in reducing significantly the number of viable tumor cells in the capsules. The results are detailed in Table 2 below.

Table 2

| In vivo inhibition of Molt-4 lymphoma growth by chemotherapeutic drugs. | | | | | |
|---|---|---|---|---|---|
| Treatment | Total volume of recovered capsules (ml) | No. of capsules | Total No. of tumor cells | No. of cells per capsule | % inhibition |
| None | 0.47 | 611 | $166 \times 10^4$ | 2717 | |
| Adriamycin (10 mg/kg) | 0.26 | 338 | $8 \times 10^4$ | 237 | 91.3 |
| BCNU (100 mg/kg) | 0.17 | 221 | $27 \times 10^4$ | 1222 | 55.0 |
| CY (200 mg/kg) | 0.45 | 585 | $10 \times 10^4$ | 171 | 93.7 |

Example 3

In the next experiment various drugs were administered in vivo according to schedules and doses typically used for screening antitumor effects of the drugs at the National Cancer Institute, Developmental Therapeutics program.

MOLT-4 human lymphoma cells were encapsulated and transplanted into peritoneum of nude mice. Doses of various drugs were applied i.v. at 1, 5 and 9 days after tumor cell transplantation. Six days after last administration, (15 days after transplantation) dramatic inhibition of tumor cell proliferation was found when Adriamycin (6 mg/kg) was used. 5-FU (60 mg/kg) was significantly less efficient.

**Table 3**

In vivo sensitivity of Molt-4 human lymphoma cells to the cytotoxic action of Adriamycin and 5-Fu.

| Treatment | Days of Treatment | No. of viable tumor cells per 1 ml of capsules | % inhibition |
|---|---|---|---|
| None | | $58.0 \times 10^6$ | |
| Cyclophosphamide (100 mg/kg) | 1, 5, 9 | $1.9 \times 10^6$ | 97 |
| Adriamycin (6 mg/kg) | 1, 5, 9 | $2.7 \times 10^6$ | 95 |
| 5-Fu (60 mg/kg) | 1, 5, 9 | $41.0 \times 10^6$ | 30 |

**Example 4**

A second human cancer cell line was also used to further analyze in vivo effects of different drugs on the growth of tumors. Encapsulated human colon adenocarcinoma (HT-29) cells were again transplanted into C57BL/6 mice and various drugs were administered after 3 days either by intravenous or by intraperitoneal injection. Animals were sacrificed after 4 days and number of cells per capsule were counted to calculate inhibition rates for the various treatment procedures. The results of these experiments are reported in Table 4 below. The encapsulated HT-29 cells were more sensitive to 5-FU than to the other drugs tested. This result correlates well with known data. In fact, 5-FU is a preferred chemotherapeutic agent for patients with gastrointestinal malignancies.

**Table 4**

Influence of the chemotherapeutic drugs on the vivo growth of encapsulated human colon adenocarcinoma HT-29 cells.

| Treatment | 4 days No. of capsules | Total No. of tumor cells | No. of cells per capsule | % inhibition |
|---|---|---|---|---|
| None | 109 | $2 \times 10^6$ | $2 \times 10^4$ | |
| 5-Fu 150 mg/kg i.p. | 99 | $0.6 \times 10^6$ | 0.6 x 104 | 70 |
| CY i.p. (250 mg/kg) | 75 | $1.1 \times 10^6$ | 1.6 x 104 | 20 |
| Adriamycin i.p. 20 mg/kg | 115 | $1.2 \times 10^6$ | 1 x 104 | 50 |
| Adriamycin i.p. 20 mg/kg | 53 | $5.8 \times 10^5$ | 1.1 x 104 | 45 |

**Example 5**

In this experiment the use of a third human tumor cell line was demonstrated. The sensitivity of human lung carcinoma A549 cells to the drugs was assessed 12 and 14 days after transplantation of encapsulated tumor cells. The treatments were applied i.v. 2, 5 and 9 days after microcapsule transplantation (Table 5). A substantial reduction of the numbers of tumor cells was observed in capsules retrieved from the treated mice in comparison to the non-treated control mice. Adriamycin and BCNU were more efficient in this respect than CY.

Table 5

In vivo susceptibility of human lung cancinoma A549 cells to chemotherapeutic drugs.

| Treatment | Dose of treatment mg/kg | 12 days | | 14 days | |
|---|---|---|---|---|---|
| | | No. of tumor cells per 1 ml of capsules | percent inhibition | No. of tumor cells per 1 ml of capsules | percent inhibition |
| None | – | $60.0 \times 10^5$ | – | $82.5 \times 10^5$ | – |
| Adriamycin | 6 | $9.0 \times 10^5$ | 85 | $13.5 \times 105$ | 84 |
| BCNU | 20 | $7.0 \times 10^5$ | 88 | $15.7 \times 105$ | 81 |
| CY | 100 | $13.5 \times 10^5$ | 78 | $39.0 \times 105$ | 53 |

Example 6

In this series of experiments, cells of a fourth human cancer type, Melanoma LOX cells, were used. LOX cells have recently been proposed as the model for experimental treatment at the NCI Development Therapeutics Program. Following i.p. inoculation of the suspension of LOX Melanoma cells into nude mice, various drugs were administered i.v. and survival of the treated mice was determined (Schoemaker et al., Proceeding American Association for Cancer Research, 26, 1302, 1985) (mice transplanted with the non-encapsulated tumor cells were considered tumor-free if they survived during 3 months of observation). In parallel experiments encapsulated LOX Melanoma cells were transferred into nude mice and drugs were inoculated i.v. 1, 5 and 9 days at the dose and schedule utilized by the Dr. Shoemaker's procedure.

The results presented in Table 6 indicate that encapsulated human Melanoma LOX cells were most sensitive to the treatment with Adriamycin, BCNU and CY. The results correlated well with the findings that these drugs were most efficient in the treatment of tumor bearing nude mice. 5-FU was the least efficient in the inhibition of the proliferation of encapsulated LOX cells and as well in the treatment of the experimental mice.

Table 6

In vivo susceptibility of Lox human melanoma cells to the chemotherapeutic drugs.

| Treatment | Dose of treatment | No. of viable tumor cells per 1 ml of capsules | % survived mice transplanted with nonencapsulated tumor cells |
|---|---|---|---|
| None | – | $160 \times 10^5$ | 0 |
| Adriamycin | 6 | $6 \times 10^5$ | 33 |
| BCNU | 20 | $2 \times 10^5$ | 50 |
| CY | 100 | $0.7 \times 10^5$ | 40 |
| 5-Fu | 60 | $29 \times 10^5$ | 0 |

Example 7

In this series of experiments, differences in sensitivity to chemotherapeutic agents were demonstrated for a lymphoma (P388) cell line and a drug-resistant variant thereof. Since drug-resistance is one of the most important causes of failure in chemotherapy, it is crucial that a predictive assay be able to distinguish between effective and non-effective drugs for particular tumors. To demonstrate the efficiency of the present invention, original P388 lymphoma cells or selected adriamycin-resistant variants of P388 lymphoma cells were inoculated i.p. into nude mice. One day later the mice received by i.v. administration Adriamycin (6 mg/kg), Vinblastin (1 mg/kg) or Cyclophosphamide (100 mg/kg). Five days after administration some of the mice were sacrificed, the microcapsules were recovered and the numbers of tumor cells inside the capsules were determined. The remaining mice received a second administration and the microcapsules were recovered from the remaining mice 3 days afterwards. The results are shown in Table 7 and correlate well with the known characteristics of P388 cell line and its variant. Neither Adriamycin nor Vinblastin in the doses administered was effective in inhibiting the growth of the drug-resistant variant. On the other hand, cyclophosphamide proved very effective (96-98 percent inhibition) in combating this cancer.

Table 7

In vivo effect of cytotoxic drugs on the growth of encapsulated P388 lymphoma cells.

| | Original P388 Cells | | | |
|---|---|---|---|---|
| | 5 days after treatment | | 5 + 3 days after treatment | |
| | No. cells/ml | % inhibition | No. cells/ml | % inhibition |
| None | $23 \times 10^6$ | — | $50 \times 10^6$ | — |
| Adriamycin | $8 \times 10^6$ | 64 | 4 x 106 | 92 |
| Vinblastin | $9 \times 10^6$ | 60 | 6 x 106 | 89 |
| CY | $0.4 \times 10^6$ | 98 | 1.3 x 106 | 97 |

| | P388 Resistant Cells | | | |
|---|---|---|---|---|
| | 5 days after treatment | | 5 + 3 days after treatment | |
| | No. cells/ml | % inhibition | No. cells/ml | % inhibition |
| None | $17 \times 10^6$ | — | 29 x 106 | — |
| Adriamycin | $18 \times 10^6$ | — | 32 x 106 | — |
| Vinblastin | $15 \times 10^6$ | 12 | 23 x 106 | 21 |
| CY | $0.4 \times 10^6$ | 98 | 1.1 x 106 | 96 |

## Claims

1. A method of assaying the sensitivity of cells to a therapeutic agent, the method comprising:
A. encapsulating the cells within at least one semipermeable membrane;
B. inoculating the encapsulated cells into a living host animal, whereby nutrients necessary to sustain the cells will pass through the semipermeable membrane;
C. exposing the encapsulated cells to the therapeutic agent, whereby the agent will be taken up by the cells through the membrane;
D. subsequently retrieving the inoculated, encapsulated cells from the host animal; and
E. measuring changes in the encapsulated cells to determine the sensitivity of the cells to the agent.

2. The method according to claim 1, wherein the therapeutic agent is of unknown efficacy and the assay further comprising comparing the results with at least one control to determine the efficacy.

3. The method according to claim 1 or claim 2, wherein the agent is a cytotoxic agent, the cells are cancer cells, and changes in cell viability are measured to determine the efficiency of the agent.

4. The method according to claim 1, 2 or 3, wherein the encapsulated cells are exposed to the therapeutic agent in vitro prior to the step of innoculating the encapsulated cells into the living host animal.

5. The method according to claim 1, 2 or 3, wherein the encapsulated cells are exposed to the therapeutic agent in vivo after the step of inoculating the encapsulated cells into the living host animal.

6. The method according to claim 5, wherein the agent is introduced into the host animal by intravenous injection, by subcutaneous injection, by suppository or orally.

7. The method according to any of claims 1 to 6, wherein the cells are encapsulated together with materials conducive to cell growth.

8. The method according to claim 7, wherein the materials include an anchorage substrate which, for example, includes collagenous protein.

9. The method according to claim 7, wherein the materials include serum components.

10. The method according to any of claims 1 to 10, wherein the encapsulation step (A) further includes forming a membrane by reaction between ionic groups on polymer chains and ionic groups of opposite charge on a water soluble gum containing the cells to form a salt bonded semipermeable membrane.

11. The method according to any of claims 1 to 10, wherein said encapsulation step (A) is effected by the steps of:
A. suspending the cells in an aqueous medium physiologically compatible therewith and containing a water-soluble gum having plural anionic moieties;
B. forming the suspension into droplets containing the cells;
C. subjecting the droplets to a solution of multivalent, physiologically compatible cations to gel the droplets as discrete, shape-retaining water-soluble temporary capsules; and

D. cross-linking surface layers of said temporary capsules to produce semipermeable membranes about said droplets by subjecting them to a polymer comprising plural cationic groups reactive with said anionic moieties.

12. The method according to claim 11, wherein the polymer is polylysine or polyornithine.

13. The method according to any of claims 1 to 12, wherein the step of measuring changes in the encapsulated cells further comprises counting viable cells with a hemacytometer and comparing such count with a control count.

14. The method according to any of claims 1 to 12, wherein the step of measuring changes in the encapsulated cells further comprises measuring the uptake of a metabolizable vital dye as an indication of the number of viable cells and comparing such measurement with a control measurement.

## Patentansprüche

1. Verfahren zum Prüfen der Empfindlichkeit von Zellen gegenüber einem therapeutischen Mittel, darin bestehend, daß

A. die Zellen in zumindest eine semipermeable Membran eingekapselt werden,

B. die eingekapselten Zellen in ein lebendes Wirtstier eingeimpft werden, wobei die zur Erhaltung der Zellen notwendigen Nährstoffe durch die semipermeable Membran hindurchgehen,

C. die eingekapselten Zellen dem therapeutischen Mittel ausgesetzt werden, wobei das Mittel von den Zellen durch die Membran hindurch aufgenommen wird,

D. anschließend die eingeimpften, eingekapselten Zellen aus dem Wirtstier zurückgewonnen werden und

E. Veränderungen in den eingekapselten Zellen zur Bestimmung der Empfindlichkeit der Zellen gegenüber dem Mittel gemessen werden.

2. Verfahren nach Anspruch 1, bei dem das therapeutische Mittel eine unbekannte Wirksamkeit hat und die Prüfung ferner darin besteht, daß die Ergebnisse mit zumindest einer Kontrolle zur Bestimmung der Wirksamkeit verglichen werden.

3. Verfahren nach Anspruch 1 oder Anspruch 2, bei dem das Mittel ein zytotoxisches Mittel ist, die Zellen Krebszellen sind und Veränderungen in der Zellenvermehrungsfähigkeit zur Bestimmung der Wirksamkeit des Mittels gemessen werden.

4. Verfahren nach Anspruch 1, 2 oder 3, bei dem die eingekapselten Zellen dem therapeutischen Mittel in vitro vor dem Schritt der Einimpfung der eingekapselten Zellen in das lebende Wirtstier ausgesetzt werden.

5. Verfahren nach Anspruch 1, 2 oder 3, bei dem die eingekapselten Zellen dem therapeutischen Mittel in vivo nach dem Schritt des Einimpfens der eingekapselten Zellen in das lebende Wirtstier ausgesetzt werden.

6. Verfahren nach Anspruch 5, bei dem das Mittel in das Wirtstier durch intravenöse Injektion, durch subkutane Injektion, durch Suppositorien oder oral eingeführt wird.

7. Verfahren nach einem der Ansprüche 1 bis 6, bei dem die Zellen zusammen mit einem Zellwachstum förderlichen Material eingekapselt werden.

8. Verfahren nach Anspruch 7, bei dem die Materialien ein Adhäsionssubstrat umfassen, das zum Beispiel ein collagenes Protein enthält.

9. Verfahren nach Anspruch 7, bei dem die Materialien Serumkomponenten enthalten.

10. Verfahren nach einem der Ansprüche 1 bis 9, bei dem der Einkapselungsschritt (A) des weiteren darin besteht, daß eine Membran durch Reaktion zwischen ionischen Gruppen an Polymerketten und ionischen Gruppen entgegengesetzter Ladung an die Zellen enthaltendem wasserlöslichem Gummi zur Bildung einer salzgebundenen semipermeablen Membran gebildet wird.

11. Verfahren nach einem der Ansprüche 1 bis 10, bei dem der Einkapselungsschritt (A) durch die Schritte ausgeführt wird, daß

A. die Zellen in einem wässerigen Medium suspendiert werden, das mit diesen physiologisch verträglich ist und ein wasserlösliches Gummi mit mehrfach anionischen Teilen enthält,

B. aus der Suspension Tröpfchen gebildet werden, die die Zellen enthalten,

C. die Tröpfchen in eine Lösung mehrwertiger, physiologisch verträglicher Kationen gegeben werden, um die Tröpfchen als diskrete, formhaltige wasserunlösliche zeitweilige Kapseln zu verfestigen, und

D. Oberflächenschichten der zeitweiligen Kapseln vernetzt werden, um semipermeable Membranen um die Tröpfchen herzustellen, indem sie einem Polymeren ausgesetzt werden, das mehrfach kationische, mit den anionischen Teilen reaktionsfähige Gruppen umfaßt.

12. Verfahren nach Anspruch 11, bei dem das Polymere Polylysin oder Polyornithin ist.

13. Verfahren nach einem der Ansprüche 1 bis 12, bei dem der Schritt des Messens von Veränderungen in den eingekapselten Zellen ferner darin besteht, daß vermehrungsfähige Zellen mit einem Hämazytometer gezählt und eine derartige Zählung mit einer Kontrollzählung verglichen wird.

14. Verfahren nach einem der Ansprüche 1 bis 12, bei dem der Schritt des Messens von Veränderungen in den eingekapselten Zellen ferner darin besteht, daß die Aufnahme eines metabolisierbaren Vitalfarbstoffs als eine Anzeige der Zahl vermehrungsfähiger Zellen gemessen und eine derartige Messung mit einer Kontrollmessung verglichen wird.

**Revendications**

1. Procédé de détermination de la sensibilité de cellules à un agent thérapeutique, ledit procédé étant caractérisé par les étapes suivantes:

A. Encapsulation des cellules dans au moins une membrane semi-perméable;

B. Inoculation des cellules encapsulées dans au moins un animal vivant hôte, les éléments nutritifs nécessaires à la sustentation des cellules passant à travers la membrane semi-perméable;

C. Exposition des cellules encapsuler à l'agent thérapeutique, l'agent étant prélevé par les cellules à travers la membrane;

D. Recherche subséquente des cellules inoculées encapsulées issu de l'animal hôte; et

E. Mesure des changements dans les cellules encapsulées pour déterminer la sensibilité des cellules à l'agent.

2. Procédé suivant la revendication 1 caractérisé par le fait que l'agent thérapeutique est d'efficacité inconnue et que la détermination comprend de plus la comparaison des résultats avec au moins un essai de référence ou de contrôle pour déterminer l'efficacité.

3. Procédé selon l'une des revendications 1 ou 2 caractérisé par le fait que l'agent est un agent cytotoxique, les cellules étant des cellule cancéreuses et les changements dans la viabilité de la cellule étant mesurée pour déterminer l'efficacité de l'agent.

4. Procédé selon l'une des revendications 1, 2 ou 3 caractérisé par le fait que les cellules encapsulées sont exposées in vitro à l'agent thérapeutique avant l'étape d'inoculation des cellules encapsulées dans l'animal vivant hôte.

5. Procédé selon l'une des revendications 1, 2 ou 3 caractérisé par le fait que les cellules encapsulées sont soumises à l'agent thérapeutique in vivo avant l'étape d'inoculation de cellules encapsulées dans l'animal vivant hôte.

6. Procédé selon la revendication 5 caractérisé par le fait que l'agent est introduit dans l'animal hôte par injection intraveineuse, par injection sous-cutanée, par suppositoire ou par voie orale.

7. Procédé selon l'une quelconque des revendications 1 à 6 caractérisé par le fait que les cellules sont encapsulées avec des substances favorisant la croissance cellulaire.

8. Procédé selon la revendication 7 caractérisé par le fait que les substances comprennent un substrat de fixation qui par exemple comprend de la protéine collagéneuse.

9. Procédé selon la revendication 7 caractérisé par le fait que les substances comprennent des composants du sérum.

10. Procédé selon l'une quelconque des revendications 1 à 9 caractérisé par le fait que l'étape d'encapsulation (A) comprend de plus la formation d'une membrane par réaction entre des groupes ioniques sur des chaînes polymères et des groupes ioniques de charge opposée sur une gomme hydrosoluble contenant les cellules pour former une membrane semi-perméable à liaison saline.

11. Procédé selon l'une quelconque des revendications 1 à 10 caractérisé par le fait que ladite étape d'encapsulation (A) est effectuée selon les étapes suivantes:

a. suspension des cellules dans un milieu aqueux physiologiquement compatible avec elle et contenant une gomme hydrosoluble présentant un certain nombre de motifs anioniques.

B. formation de la suspension en gouttelettes contenant les cellules;

C. soumission des gouttelettes à une solution de cations multivalents et physiologiquement compatibles pour gélifier les gouttelettes sous forme de capsules temporaires, discrètes, insolubles dans l'eau et retenant leur forme; et

D. réticulation des couches de surface desdites capsules temporaires pour former des membranes semi-perméables autour desdites gouttelettes en les soumettant à un polymère comprenant un certain nombre de groupes cationiques réactifs avec lesdits motifs anioniques.

12. Procédé selon la revendication 11 caractérisé par le fait que le polymère est de la polylysine ou de la polyornithine.

13. Procédé selon l'une quelconque des revendications 1 à 12 caractérisé par le fait que l'étape de mesure des changements des cellules encapsulées comprend de plus le comptage des cellules viables avec un hémacytomètre et la comparaison de ce comptage avec un comptage de référence ou de contrôle.

14. Procédé selon l'une quelconque des revendications 1 à 12 caractérisé par le fait que l'étape des mesures des changements dans les cellules encapsulées comprend de plus la mesure de la prise d'un colorant vital métabolisable comme indication du nombre de cellules viables et la comparaison d'une telle mesure avec une mesure de comparaison ou de contrôle.